# EUROPEAN PATENT SPECIFICATION

(11) **EP 2 385 821 B1**
(45) Date of publication and mention of the grant of the patent: **31.08.2016**
(21) Application number: 10703346.6
(22) Date of filing: 08.01.2010
(51) Int. Cl.: A61K 9/02, A61K 47/10, A61K 31/4174

(54) **IMPROVED PHARMACEUTICAL FORMULATION**
VERBESSERTE PHARMAZEUTISCHE FORMULIERUNG
FORMULATION PHARMACEUTIQUE AMÉLIORÉE

(30) Priority: 09.01.2009 HU 0900010; 07.01.2010 HU 1000006
(43) Date of publication of application: 16.11.2011
(73) Proprietor: Egis Gyógyszergyár Zrt., 1106 Budapest (HU)
(72) Inventor: MIKULASIK, Endre, H-9783 Egyházasrádóc (HU); SZAKALY, Péter, H-9900 Körmend (HU)
(74) Representative: Stolmár & Partner Patentanwälte PartG mbB
(86) International application number: PCT/HU2010/000003
(87) International publication number: WO 2010/079373

(56) References cited:
- EP-A1- 0 271 882
- EP-A1- 0 770 384
- EP-A2- 0 404 376
- WO-A1-97/00670
- WO-A2-2007/079389
- US-A- 4 853 211
- US-A1- 2005 276 836
- PHILLIPS A J: "Treatment of non-albicans Candida vaginitis with amphotericin B vaginal suppositories" AMERICAN JOURNAL OF OBSTETRICS & GYNECOLOGY, MOSBY, ST LOUIS, MO, US LNKD- DOI:10.1016/J.AJOG.2005.03.034, vol. 192, no. 6, 1 June 2005 (2005-06-01), pages 2009-2012, XP004937211 ISSN: 0002-9378

## Description

### Field of the invention

The invention relates to a vaginal suppository with improved efficacy containing an antifungal active ingredient, wherein said active ingredient is present simultaneously in dissolved and suspended form in a suppository base containing polyethylene-glycol and polysorbate. The ratio of the suspended and dissolved active ingredient in the suppository is reproducible and said ratio does not change during the storage. A further object of the present invention is a process for manufacturing said pharmaceutical formulations.

### Technical background of the invention

The most widespread vaginal diseases are several kinds of fungal infections. In the treatment of these infections imidazole antifungals are generally used. Several pharmaceutical active ingredients are known for use in this therapy.

There are some disadvantages when using tablets for oral administration containing imidazole-type active ingredients. Absorption and distribution of the active ingredient takes a long time until the area of the infection by the systemic circulation is reached. In order to achieve sufficient blood concentration for treating said infections, high doses are necessary. The high amount of the active ingredient causes difficulties during formulation and additionally it is inconvenient for the patient to take large tablets. Because of the fast decomposition of the active ingredient the treatment is to be carried out several times a day or it may last several days. The poor solubility of the active ingredient and the large dose may cause several undesired side effects.

Several creams, ointments, solutions and emulsions used for local administration are known. In case of these formulations the active ingredient gets through directly to the area of infection, but the formulations are soon liquified thus the required amount of the active ingredient is not present at the area of the infection and does not exhibit its effect through the desired period, and this can be inconvenient for the patient.

Other therapeutic options for treating fungal infections are vaginal suppositories containing an imidazole derivative. The vaginal suppositories should meet some general requirements such as that the active ingredient must be present at the infected area in suitable form, in an appropriate amount and concentration for a sufficient period in order to exhibit the required effect. The large number of publications in this field of therapy and the high variety of the marketed antifungal suppositories prove that the actually applied suppositories are able to satisfy only some of the above mentioned requirements.

The known suppositories contain the active ingredient in suspended form formulated in a high mucosa-adhesive oleaginous base.

The publication of Dellenbach et al. describes studies with oleaginous suppositories used for the treatment of Vulvovaginal candidiasis, wherein the effectiveness of sertarconazole containing suppository is compared with econazole containing suppositories (Dellenbach P, Thomas JL, Guerin V, et al: Topical treatment of vaginal candidosis with sertaconazole and econazole sustained-release suppositories. Int J Gynecol Obstet 2000; 71:S47-S52)*.* 310 women with symptoms and signs of vulvovaginitis were enrolled in this randomized study. There was a trend towards a better efficacy of sertaconazole because the mycological recurrence rate was lower than when using econazole. The patients were treated with a 300-mg sertaconazole suppository and a 150-mg econazole sustained release suppository. The results were evaluated 1 week after treatment and patients whose treatment was unsuccessfull received a second suppository. 96 per cent of the patients were infected by *Candida albicans.* 105 women were not clinically cured after 1 week (49 in the sertaconazole group and 56 in the econazole group) and received a second treatment

The second administration was necessary because the recurrence of symptoms occured in high rate after the single dose application independently from the active ingredient used or its efficacy. The main reason of this problem is mostly the suppository base, which can not penetrate into the narrow folds of the mucosal membrane at the margin of uterus and vagina. Therefore some active pathogens can remain on this surface and are able to cause a high percentage of mycological recurrence.

Polyethylene-glycols have been used as suppository base for a long time. As it is known the advantages of polyethylene glycol base suppositories over oil base suppositories are the more favourable adhesion and extension properties. These facts are presented in Example 2 (Comparative mold experience 2.1, 2.2).

A further important requirement towards intravaginal suppositories is that they should reach all parts of the wall of vagina, including the narrow folds of the mucosal membrane. This can provide the development of the effect on the entire surface of the vagina.

Suppositories are known, in which the active ingredient is dissolved or suspended, depending on the character of the active ingredient.

The dissolved active ingredient promptly exhibits its effect but due to the poor adhesion capacity and viscosity of the solution it only can difficultly adhere on the wall of vagina and leaves the infected area soon, therefore the period of the drug is shortened and the probability of the recurrence increased. A further disadvantage of the dissolved active ingredient containing suppository is that the administration is to be repeated many times during the therapy.

Econazol (as the antifungal imidazol derivatives generally) is poorly soluble in water and oil or in the polyethylene-glycol suppository base. Therefore such active ingredients are used in the form of a suspension. Said suspension meets the therapeutic requirements because the particles adhere on the mucosal membrane of the vagina and provide the therapeutically effective amount (i.e. microgram) of the active ingredient in an autoretard manner. The effect of the suppositories, which consits only of the suspended active ingredient is long-lasting, but the effect develops slowly and there is a lower probability for the active ingredient to reach the infected area in the suitable concentration.

Pharmaceutical compositions, which show the advantageous properties of the two dosage forms and are used simultaneously, are not known in prior art.

It is known that some compositions containing polyethylene-glycol as vehicle, contain the active ingredient partially in dissolved and partially in precipitated form. But in case of these compositions the active ingredient precipitates during the cooling of the composition in a random manner and therefore the amount, particle size and dispersion of the precipitated active ingredient are occasional. Such composition are described in Example 3 of US2005/276836 A1, in Example 4 of US4853211A1, Example 7 of WO97/00670A1 and in page 2010, column 2, paragraph 3 of Phillips A. J., Am. J. Obst. & Gynec. (2005) vol. 192, no.6, p. 200p-2012. The ratio of the precipitated and dissolved active ingredient is random and not-reproducible; moreover the ratio can alter during storage

The object of the present invention is to provide a pharmaceutical formulation which can ensure that the active ingredient is present at the inflected area in suitable form, appropriate amount and concentration for a sufficient period in order to exhibit the desired effect and the active ingredient is able to reach all parts of the wall of vagina, including the narrow folds of the mucosal membrane.

### Summary of the invention

The present invention is based on the recognition that the advantageous properties of the two dosage forms can be combined in one composition

It has been surprisingly found that the composition of the present invention can be formulated reproducibly in such way that a pre-determined portion of the active ingredient is dissolved and the remaining portion is in suspended form. The dosage form of the present invention has more favourable properties regarding the development of the therapeutic effect than the compositions which contain the active ingredient solely in suspended or in dissolved form. This observation can be explained by the fact that the dissolved active ingredient exhibits its effect rapidly and can penetrate much deeper and the suspended part of active ingredient can maintain this effect for a long period of time. This effect is enhanced by the viscosity and adhesion of the vehicle.

The present invention relates to a vaginal suppository containing an antifungal active ingredient simultaneously in suspended and dissolved form, wherein the ratio of the suspended and dissolved active ingredient is reproducible and does not change during the storage. The ratio of the suspended and dissolved active ingredient can vary depending on the character of the active ingredient.

A further aspect of the invention is a process for the preparation of suppositories wherein a pre-determined portion of the active ingredient is dissolved in a mixture containingpolyethylene-glycol and other excipients, thereafter the mixture is cooled and the remaining portion of the active ingredient is suspended in the mixture in solid form

### Detailed description of the invention

The present invention relates to a vaginal suppository containing an antifungal active ingredient simultaneously in suspended and dissolved form, wherein the ratio of the suspended and dissolved active ingredient is reproducible and does not change during the storage.

The ratio of the suspended and dissolved active ingredient can vary depending on the character of the active ingredient.

Another aspect of the present invention is a suppository wherein a pre-determined portion of the active ingredient is dissolved in a mixture containing polyethylene-glycol and other excipients and the remaining portion of the active ingredient is suspended in the mixture in solid form. The ratio of the suspended and dissolved active ingredient can vary depending on the properties of the active ingredient.

Another aspect of the present invention is a process for the preparation of a polyethylene-glycol base suppository, which contains an antifungal active ingredient in dissolved and suspended form. The suppository of the present invention is prepared by dissolving a pre-determined portion of the active ingredient in a polyethylene component containing suppository base, cooling the mixture, thereafter suspending the remaining portion of the active ingredient in the mixture.

The melting point of the suppository base of the present invention is approximately 48-52 °C therefore it does not dissolve at body temperature. However it is mildly hygroscopic and soluble in water therefore it liquifies, forms a viscous blend and is dissolved already under the effect of minimal vaginal fluid..

After liquefying and during dissolution the suspended portion of the active ingredient is set free and the active ingredient particles disperse on the wall of the vagina thus developing the first effective front.. The suppository of the present invention contains a second effective front, too. This front is developed by dissolving the suppository base in the body fluids, which penetrates into the closed spaces and between the narrow folds of the mucosal membrane. During dissolution the polyethylene-glycol is diluted by water, therefore due to the solubility decrease the active ingredient precipitates in form of microparticles at the place of the dissolution (i.e. between the narrow folds of the mucosal membrane), thus developing the second effective front.

The polyethylene-glycol and polysorbate based suppository composition of the present invention contains preferably an active ingredient selected from econazole, econazole-nitrate, bifonazole, butoconazole, clotrimazole, croconazole, fenticonazole, ketoconazole, miconazole, omoconazole, oxiconazole, sulconazole and/or tioconazole, more preferably econazole and/or econazole-nitrate or pharmaceutical acceptable salt thereof as antifungal active ingredient. The pharmaceutical composition of the present invention contains preferably 150 to 300 mg econazole and/or econazole-nitrate.

The pharmaceutical composition of the present invention may contain a polysorbate as excipient. The polysorbate ingredient of the suppository may be Polysorbate 20, Polysorbate 60 and/or Polysorbate 80, preferably Polysorbate 60. The polysorbate ingredient of the suppository is present preferably in 1.5 per cent amount by weight.

Polyethylene-glycol polymer ingredient of the suppository may be any of the known polyethylene-glycols with molecular weight of 100 to 4000, preferably Macrogol 1500, Macrogol 1000 and/or Macrogol stearate. The known polyethylene-glycols with molecular weight of 100 to 4000 can be the for example the follows: PEG 100, PEG 200, PEG 400, PEG 540, PEG 600, PEG 900, PEG 1000, PEG 1450, PEG 1540, PEG 2000, PEG 3000, PEG 3350, PEG 4000. Commercially available polyethylene-glycol containing suppository bases can also be used as a suppository base of the present invention.

The amount of the suppository base is preferably a ten-fold amount of the active ingredient.

A further aspect of the present invention is a process for the preparation of suppository, wherein the suppository mixture is poured; preferably the melted mixture is poured into a pre-covered suppository mold cavity. This shell can be used as the final package of the suppository. The suppository of the present invention is de preferably prepared by dissolving a pre-determined portion of the active ingredient, preferably 20 per cent in a polyethylene glycol containing suppository base. The polysorbate is already dissolved in the polyethylene glycol. After dissolving the active ingredient the mixture is cooled. The next step is suspending the remaining portion of the active ingredient in the mixture.

The main advantage of the present invention is that the composition of the suppository makes possible for the active ingredient not only to possess the antifungal or anti-inflammatory effect but also to penetrate and being absorbed between the narrow folds of the mucosal membrane at the margin of uterus and vagina, thus effectively decreasing the development of the recurrence.

The invention is further elucidated by means of following Examples without restricting the scope of the present invention to the Examples.

### Example 1. : Investigation of the active ingredient dispersion

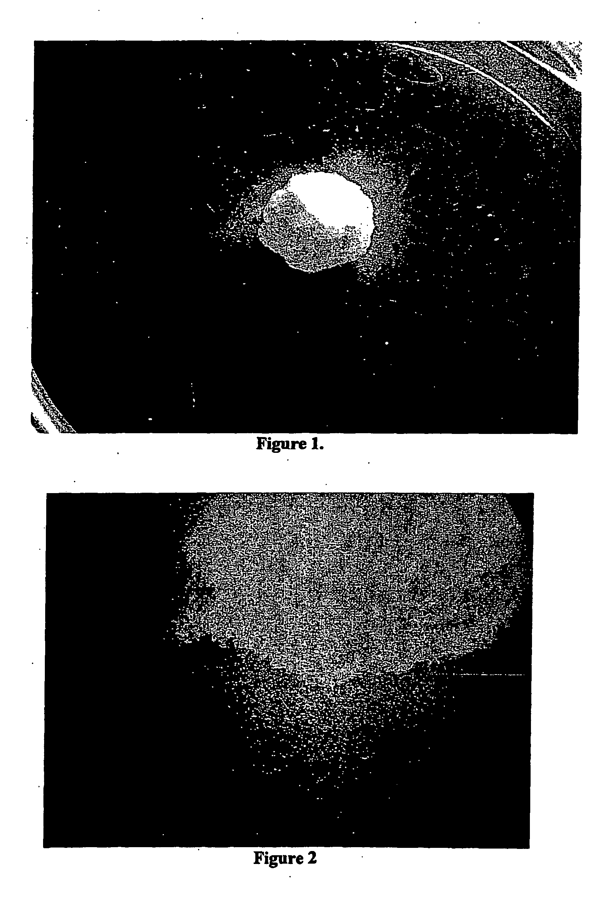

The mechanism of the active ingredient distribution is shown on Figure 1 and 2. A slice of the suppository prepared according to Example 3 was placed into body-temperature water in a Petri dish and the segment was allowed to stand for 5 minutes. In Figure 1 the non-melted segment can be seen. Around the non-melted segment there is a white ring which is the suspended active ingredient set free. Around the white ring there can be seen the radial band of the micro crystals which precipitated from the solution along the decrease of concentration due to dilution.

The microscopic structure of the micro crystal band can be seen more closely in Figure 2. Said Figure 2. shows the matrix, the suspended active ingredient particles, which were suspended in the matrix and fell out from the matrix after melting and also the micro crystals which precipitated from the solution along the decrease of concentration.

### Example 2.: Comparative example

In order to demonstrate, that the polyethylene-glycol base suppository has more favourable adhesion and extension properties a comparison with oleaginous base suppositories some model-experiments were performed. The results of said experiments are summarised below.

### 2.1. Slide-down test

Comparative experiments were preformed to study the adhesion properties of the mucosa membrane with a commercially available oleaginous-base suppository and with the suppository of Example 3. The aim of the experiment was to establish whether the oleaginous or the polyethylene-glycol base suppository has better mucosa-adhesive properties *in vitro.*

3,3 g average-weight suppositories were poured from both mixtures and the mixture were marked by crystal violet in order to facilitate observation.

A gelatine layer of 1 mm thickness poured into a 19 mm Petri dish served as mucosa model. The surface of the gelatine was spread over 20 ml of 0.5 per cent methocell mucosa directly before the experiment. The experiments were performed at body temperature, 37 °C.

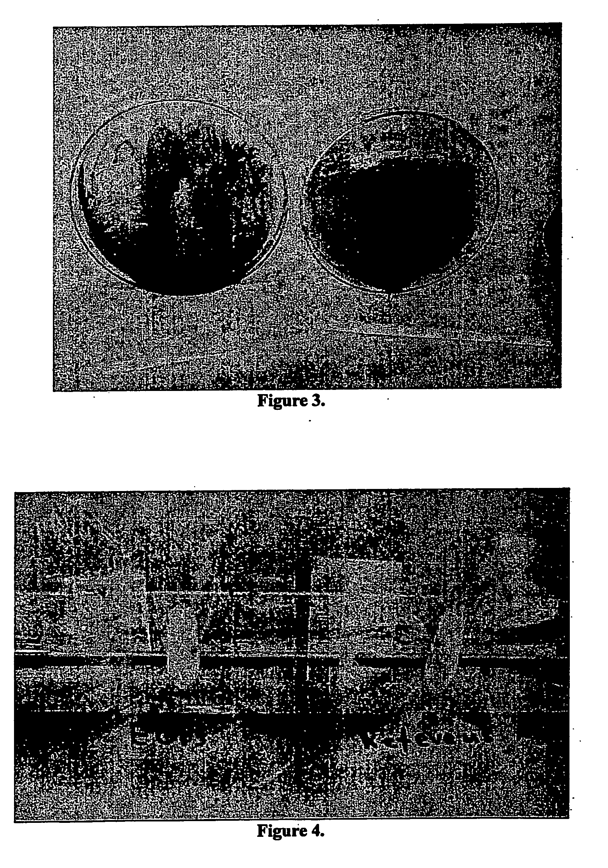

At 37 °C the oleaginous suppository was melt and the suppository of the present invention was liquefied by addition of 1,5 ml water. The mixtures were smoothly spread over the models horizontally so that the entire surface of the model was covered. After 5 minutes the models were positioned vertically and the results were evaluated after 1 minute. Figure 3. shows the results: the oleaginous base suppository can be seen in the left side and the suppository of the present invention can bee seen in the right side of the Figure 3.

### Evaluation:

It can be concluded that the oleaginous base suppository slid down from the surface and flow back on the top of the model, the adhesion of the suppository was not satisfactory. The mixture of the present invention was adhered at the whole surface of the model and no flow-back was observed. This mixture had more favourable adhesion properties.

### 2. 2. Vaginal-model experiment

To demonstrate the behaviour of the suppositories in the vagina the dialysis-membrane experiment was except that said model was used for direct observation instead of measuring the active ingredient liberation *(*Yamazaki M, Itoh S, Sasaki N, Tanabe K, Uchiyama M. Modification of the dialysis membrane method for drug release from suppositories. Pharm Res 1993; 10: 927-929*).*

The oleaginous base suppository and the suppository of the present invention were placed into the membrane according to Figure 4.

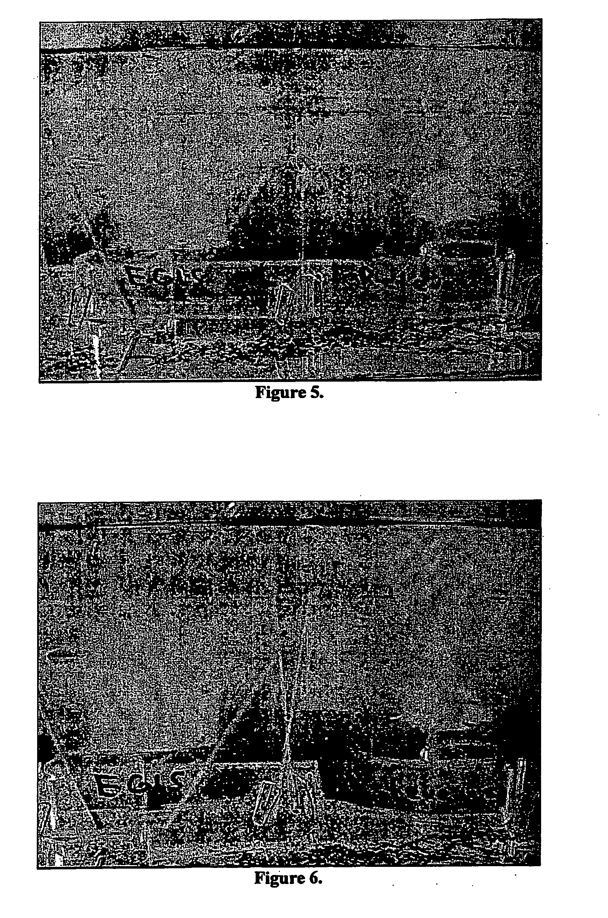

The membranes were studied in a 37 °Cwater bath. After 10 minutes the oleaginous suppository melted, while the polyethylene base suppository started to dissolve due to the low humidity, which could penetrate through to the membrane, as it can be seen in Figure 5.

Figure 6 shows the condition of the suppositories after 1 hour. It can be seen that the oleaginous suppository completely melted and the mass of the suppository was pressed up to the opening along the folds of the membrane due to the pressure of the water (tissue pressure in the organisms). The suppository of the present invention did not completely melt, but the suspension penetrated into the narrow folds of the membrane despite of the pressure of the water and filled in the available place.

### Conclusions:

Our experiments support the previous findings that the polyethylene-glycol base suppositories have much more favourable adhesion and expansion properties in comparisone with oleaginous base suppositories.

### Example 3.: Econazole containing vaginal suppository

The following composition were used for prepare one suppository with 3300 g. :

| | |
|---|---|
| Econazole | 300 mg |
| Base: | 3000 mg |

**Composition of the base:**

| | |
|---|---|
| Polysorbate 60 | 1,5% |
| Macrogol 1500 | 98,5% |

### Formulation of the suppository (~1 kg/300 pieces):

985 g Macrogol 1500 is melted and 15 g Polysorbate 60 is dissolved in the molten mass. Subsequently the temperature is adjusted to 60 degree Celsius and 900 g from the mixture is weighted. 18 g of econazole are dissolved in the mixture under continuously stirring (this is the 20 per cent of the total econazole content). After total dissolution the mixture is adjusted to 55 °C and the the remaining 72 g econazol is suspended in the mixture. The mixture is continuously stirred until pouring.

The suppository mass is poured into Erweka mold type pre-covered PE cased PVC mold cavity and after congealing the mold is heat sealed

### Example 4.: Econazole nitrate containing vaginal suppository

The composition of the suppository and the process for manufacturing the suppository are the same as described in Example 3. In case of using econazole nitrate 85 per cent (85 g) of the active ingredient are dissolved and 15 per cent (15 g) of the active ingredient is suspended in the base.

### Example 5.: Miconazole containing vaginal suppository

The composition of the suppository and the process for manufacturing the suppository are the same as it is decribed in Example 3. In case of using mikonazole 66 per cent (66,7 g) of the active ingredient is dissolved and 33 per cent (33,3 g) of the active ingredient are suspended in the base.

### Example 6.: Miconazole nitrate containing vaginal suppository

The composition of the suppository and the process for manufacturing the suppository are the same as described in Example 3. In case of using miconazole nitrate 70 per cent (70 g) of the active ingredient are dissolved and 30 per cent (30 g) of the active ingredient are suspended in the base.

### Example 7.: Ketoconazole containing vaginal suppository

The composition of the suppository and the process for manufacturing the suppository are the same as described in Example 3. In case of using ketoconazole 80 per cent (80 g) of the active ingredient are dissolved and 20 per cent (20 g) of the active ingredient are suspended in the base.

### Example 8.: Metronidazole containing vaginal suppository

The composition of the suppository and the process for manufacturing the suppository are the same as described in Example 3. In case of using metronidazole 90 per cent (90 g) of the active ingredient are dissolved and 10 per cent (10 g) of the active ingredient are suspended in the base.

## Claims

1. Vaginal suppository containing an antifungal or anti-inflammatory active ingredient simultaneously in suspended and dissolved form, wherein the ratio of the suspended and dissolved active ingredient is reproducible and does not change during the storage, wherein the composition can be formulated reproducibly in such a way that a pre-determined portion of the active ingredient is dissolved and the remaining portion is in suspended form, the vaginal suppository obtainable by dissolving a predetermined portion of the active ingredient in a mixture of polyethylene-glycol and other excipients dissolved therein, cooling the mixture and suspending the remaining portion of active ingredient therein.

2. Vaginal suppository according to claim 1, wherein the antifungal active ingredient is econazole, econazole-nitrate, bifonazole, butoconazole, clotrimazole, croconazole, fenticonazole, ketoconazole, metronidazole, miconazole, miconazole nitrate, omoconazole, oxiconazole, sulconazole and/or tioconazole, preferably econazole and/or econazole-nitrate or pharmaceutical acceptable salt thereof.

3. Vaginal suppository according to claim 1, wherein the suppository contains 150 to 300 mg econazole and/or econazole nitrate.

4. Vaginal suppository according to claim 1, which contains polysorbate in the suppository mass as excipient, and the polysorbate ingredient is Polysorbate 20, Polysorbate 60 and/or Polysorbate 80, preferably Polysorbate 60.

5. Vaginal suppository according to claim 1, wherein the polyethylene-glycol polymer ingredient of the suppository is a polyethylene-glycol with molecular weight of 100 to 4000, preferably Macrogol 1500, Macrogol 1000 and/or Macrogol stearate.

6. Vaginal suppository according to claim 1, which contains ten-fold amount of the active ingredient as suppository base.

7. Vaginal suppository base according to claim 4, which contains 1.5 per cent of polysorbate, preferably Polysorbate 60.

8. Process for preparing vaginal suppository according to claim 1, which comprises pouring the suppository mass, preferably pouring the melted mixture into a pre-covered suppository mold cavity.

9. Process for manufacturing vaginal suppository containing an antifugal or anti-inflammatory active ingredient simultaneously in suspended and dissolved form thereof which comprises dissolving a pre-determinated portion of the active ingredient in a mixture of polyethylene-glycol and other excipients dissolved therein, cooling the mixture and suspending the remaining portion of active ingredient therein.

## Patentansprüche

1. Vaginalzäpfchen, enthaltend einen antimykotischen oder antiinflammatorischen Wirkstoff gleichzeitig in suspendierter und gelöster Form, wobei das Verhältnis zwischen sich in Suspension befindlichem und gelöstem Wirkstoff reproduzierbar ist und sich während der Lagerung nicht ändert, wobei die Zusammensetzung reproduzierbar so formuliert werden kann, dass ein vordefinierter Anteil des Wirkstoffs gelöst ist und der übrige Anteil in suspendierter Form ist, das Vaginalzäpfchen erhältlich durch Lösen eines vordefinierten Anteils an Wirkstoff in einer Mischung aus Polyethylen-Glykol und anderen darin gelösten Arzneiträgern, Abkühlen der Mischung und In-Suspension-Bringen des übrigen Anteils an Wirkstoff darin.

2. Vaginalzäpfchen gemäß Anspruch 1, wobei der antimykotische Wirkstoff Econazol, Econazolnitrat, Bifonazol, Butoconazol, Clotrimazol, Croconazol, Fenticonazol, Ketoconazol, Metronidazol, Miconazol, Miconazolnitrat, Omoconazol, Oxiconazol, Sulconazol und/oder Tioconazol ist, bevorzugt Econazol und/oder Econazolnitrat oder ein pharmazeutisch verträgliches Salz davon.

3. Vaginalzäpfchen gemäß Anspruch 1, wobei das Zäpfchen 150-300 mg Econazol und/oder Econazolnitrat enthält.

4. Vaginalzäpfchen gemäß Anspruch 1, welches Polysorbat in der Zäpfchenmasse als Arzneiträger enthält, und der Polysorbat-Bestandteil Polysorbat 20, Polysorbat 60 und/oder Polysorbat 80 ist, bevorzugt Polysorbat 60.

5. Vaginalzäpfchen gemäß Anspruch 1, wobei der Polyethylen-Glykol-Polymerbestandteil des Zäpfchens ein Polyethylen-Glykol mit einem Molekulargewicht von 100-4000 ist, bevorzugt Macrogol 1500, Macrogol 1000 und/oder Macrogolstereat.

6. Vaginalzäpfchen gemäß Anspruch 1, welches die zehnfache Menge an Wirkstoff als Zäpfchenbasis enthält.

7. Vaginalzäpfchen gemäß Anspruch 4, welches 1,5 % Polysorbat enthält, bevorzugt Polysorbat 60.

8. Verfahren zur Herstellung eines Vaginalzäpfchen gemäß Anspruch 1, welches umfasst Eingießen der Zäpfchenmasse, bevorzugt Eingießen der geschmolzenen Mischung in einen vorher abgedeckten Hohlraum einer Zäpfchen-Form.

9. Verfahren zur Herstellung von Vaginalzäpfchen enthaltend einen antimykotischen oder antiinflammatorischen Wirkstoff gleichzeitig in suspendierter und gelöster Form davon, welches umfasst Lösen eines vordefinierten Anteils an Wirkstoff in einer Mischung aus Polyethylen-Glykol und anderen darin gelösten Arzneiträgern, Abkühlen der Mischung und In-Suspension-Bringen des übrigen Anteils des Wirkstoff darin.

## Revendications

1. Suppositoire vaginal contenant un ingrédient actif antifongique ou anti-inflammatoire simultanément sous forme suspendue et dissoute, dans lequel le rapport de l'ingrédient actif suspendu et dissous est reproductible et ne change pas au cours du stockage, dans lequel la composition peut être formulée de manière reproductible de telle sorte qu'une portion prédéterminée de l'ingrédient actif est dissoute et la portion restante est sous forme suspendue, le suppositoire vaginal pouvant être obtenu en dissolvant une portion prédéterminée de l'ingrédient actif dans un mélange de polyéthylène-glycol et d'autres excipients dissous en son sein, refroidissant le mélange et mettant en suspension la portion restante d'ingrédient actif en son sein.

2. Suppositoire vaginal selon la revendication 1, dans lequel l'ingrédient actif antifongique est l'éconazole, le nitrate d'éconazole, le bifonazole, le butoconazole, le clotrimazole, le croconazole, le fenticonazole, le kétoconazole, le métronidazole, le miconazole, le nitrate de miconazole, l'omoconazole, l'oxiconazole, le sulconazole et/ou le tioconazole, de préférence l'éconazole et/ou le nitrate d'éconazole ou un sel pharmaceutique acceptable de celui-ci.

3. Suppositoire vaginal selon la revendication 1, dans lequel le suppositoire contient 150 à 300 mg d'éconazole et/ou de nitrate d'éconazole.

4. Suppositoire vaginal selon la revendication 1, qui contient du polysorbate dans la masse de suppositoire comme excipient, et l'ingrédient de polysorbate est le Polysorbate 20, le Polysorbate 60 et/ou le Polysorbate 80, de préférence le Polysorbate 60.

5. Suppositoire vaginal selon la revendication 1, dans lequel l'ingrédient polymère de polyéthylène-glycol du suppositoire est un polyéthylène-glycol avec un poids moléculaire de 100 à 4000, de préférence le Macrogol 1500, le Macrogol 1000 et/ou le stéarate de Macrogol.

6. Suppositoire vaginal selon la revendication 1, qui contient une quantité décuple de l'ingrédient actif comme base de suppositoire.

7. Base de suppositoire vaginal selon la revendication 4, qui contient 1,5 pour cent de polysorbate, de préférence de Polysorbate 60.

8. Procédé de préparation d'un suppositoire vaginal selon la revendication 1, qui comprend verser la masse de suppositoire, de préférence verser le mélange fondu dans une cavité de moule de suppositoire couverte au préalable.

9. Procédé de fabrication d'un suppositoire vaginal contenant un ingrédient actif antifongique ou anti-inflammatoire simultanément sous forme suspendue et dissoute de celui-ci qui comprend dissoudre une portion prédéterminée de l'ingrédient actif dans un mélange de polyéthylène-glycol et d'autres excipients dissous en son sein, refroidir le mélange et mettre en suspension la portion restante d'ingrédient actif en son sein.
